# EUROPEAN PATENT APPLICATION

(11) **EP 4 595 897 A1**
(43) Date of publication of application: **06.08.2025**
(21) Application number: 24155195.1
(22) Date of filing: 01.02.2024
(51) Int. Cl.: A61B 8/08, A61B 8/00

(54) **APPARATUS AND METHOD FOR PERFORMING DIAGNOSTIC IMAGING EXAMINATIONS**

(71) Applicant: Esaote S.p.A., 16152 Genova (IT)
(72) Inventor: Guraschi, Nicola, 16142 Genova (IT); Zini, Luca, 16147 Genova (IT)
(74) Representative: Bessi, Lorenzo

(57) **Abstract**

An apparatus for performing diagnostic imaging examinations comprises:
an image acquisition unit,
at least one scanning controller comprising an electronic unit for operating and controlling the execution of an image acquisition protocols by means of the said image acquisition unit and for carrying out image processing on at least part of the acquired images;
output units comprising at least a display for displaying one or more of the acquired images or of processed images;
said at least one electronic unit of the said scanning controller being provided with a memory in which reference images of a sequence of said reference images acquired in following an ultrasound image acquisition protocol are stored, the said reference images being univocally associated to one or more anatomical landmarks which are present on each of them;
said at least one electronic processing unit of the said scanning controller being provided in combination with a computer program containing instructions which when executed by the said electronic unit renders the said unit able to:
run an automatic landmark detector which carries out an automatic landmark detection within each image acquired by the image acquisition unit executing said image acquisition protocol;
compare one or more landmarks detected on each one of the acquired images with the sequence of reference images and the one or more landmarks identified on the said reference images;
display each of the reference images on a display and highlight the reference images in which the one or more landmark is corresponding to the one or more landmark in the acquired images;
set the image acquisition protocol as correctly completed and
memorize the acquired images as being obtained by a correct scan for further use.

## Description

### FIELD OF THE INVENTION

The present invention relates to an apparatus for performing diagnostic imaging examinations comprising an image acquisition unit, at least one electronic unit for operating and controlling image acquisition protocols and for carrying out image processing and a generator comprised within said image acquisition unit, a user control interface comprising input organs for setting signal acquisition and processing procedures for obtaining images and for setting display modes and output organs comprising at least a display for displaying one or more of the following items consisting in graphic interface tools for communicating with a user, apparatus status information and/or status information's about an image acquisition workflow, one or more of the acquired images or of processed images.

### STATE OF THE ART

Such apparatuses are known and widely used within the diagnostic clinical practice, and up to now they have been the object of continual improvements, considerably increasing their operational potentialities.

On the other hand, such considerable increase of the operational potentialities caused the complexity to considerably increase, at least as regards the use of such apparatuses by a user.

Generally, each individual apparatus is used for different types of clinical examinations, each type being performed by a category of skilled people that use only a part of the functionalities of the apparatus in relation to their own skills while other functionalities are kept for other categories of skilled people.

A single skilled user can also operate within an environment composed of multiple types of clinical examinations, often limited only to specific organs or anatomical districts to be examined. The scanning of an anatomical district or of an organ by ultrasound is often complicated by the fact that hard tissues, such as bones, are highly echogenic, this means that this kind of tissues does not allow the ultrasound signals to pass through reaching areas which are behind these tissues.

A non-limiting specific example of the above cases is the scanning with ultrasound of the liver. Indeed, liver ultrasound imaging is a complex examination because part of the liver is covered by the rib cage. Ultrasound clinical scanning protocols have been therefore developed and are available at the state of the art ensuring that, if followed by a radiologist governing an ultrasound probe, a complete coverage of the liver will be obtained. For doing this, different, so-called, windows, in the structure of the ribcage are used through which the transmitted ultrasound beams can arrive at the liver and the reflected ultrasound beams can reach back the receiving transducers of the probe.

Currently available scanning protocols for ultrasound imaging of the liver include precise scan sequences identified by anatomical landmarks that need to be detected during the scan in the sequence of images produced by the said scan. According to the prior art methods, checking the correctness of the scanning action by correctly applying a scanning protocol relies completely on operator accuracy in defining probe trajectory and ability to detect anatomical landmarks.

Document WO2016189313 discloses a system and a method for providing feedback to the machine operator during the imaging procedure to facilitate image collection and gathering of statistics on the imaging protocol and help to maximise adherence to imaging protocols.

The feedback comprises an indication of the quality of acquired images, for example by the display of an indicator providing feedback on the acquired image quality or the provision of an audible signal. This enables the machine operator to rapidly identify captured images which do not satisfy one or more of the quality metrics.

This document goes in the direction as it provides a solution that allows to reduce operator dependency, however the therein disclosed method and system are mainly concerned with quality of scanned images rather than the correct fulfilment of a scanning protocol.

### SUMMARY OF THE INVENTION

The present disclosure aims at overcoming said prior art drawbacks by providing an apparatus and a method as defined hereinbefore which provide a reliable and easy-to-use tool for ensuring that a correct ultrasound imaging scan of an organ, and particularly of the liver, is carried out.

Furthermore, the present disclosure aims at providing a tool for overcoming the drawback of the prior art apparatus and methods which can be easily adapted to the imaging of different anatomic districts or organs.

According to an embodiment, an apparatus for performing diagnostic imaging examinations comprises:
an image acquisition unit,
at least one scanning controller comprising an electronic unit for operating and controlling the execution of an image acquisition protocol by means of said image acquisition unit and for carrying out image processing on at least part of the acquired images;
output units comprising at least a display for displaying one or more of the acquired images or of processed images;
said at least one electronic unit of the said scanning controller being provided with a memory in which reference images of a sequence of said reference images acquired by following an ultrasound image acquisition protocol are stored, the said reference images being univocally associated to one or more anatomical landmarks which are present on each of them;
said at least one electronic processing unit of the said scanning controller being provided in combination with a computer program containing instructions which when executed by said electronic unit renders said unit able to:
   run an automatic landmark detector which carries out an automatic landmark detection within each image acquired by the image acquisition unit executing said image acquisition protocol;
   compare one or more landmarks detected on each one of the acquired images with the sequence of reference images and the one or more landmarks identified on the said reference images;
   display each of the reference images on a display and highlight the reference images in which the one or more landmark is corresponding to the one or more landmark in the acquired images;
   setting the image acquisition protocol as correctly completed and
   memorize the acquired images as being obtained by a correct scan for further use.

According to an embodiment the apparatus may comprise:
a generator of a user interface which is comprised within said image acquisition unit, said user interface comprising:
one or more input units for setting signal acquisition and processing procedures for obtaining images and
for setting display modes and
one or more output units comprising at least a display for displaying one or more of the following items consisting in graphic interface tools for communicating with a user, apparatus status information and/or status information about an image acquisition workflow;
said at least one electronic unit of the scanning controller being provided in combination with a computer program containing instructions which when executed by the said electronic unit controls the display to
display at least some of the saved reference images;
highlight one after the other the reference images displayed when an acquired image has been found in which at least a landmark is detected corresponding to at least one landmark represented in respectively one of the said reference images.
terminating the image acquisition by signalling that the image acquisition protocol has been executed correctly when for each reference image an acquired image has been found having at least one landmark which are identical.

According to an improved embodiment, the system is further provided with an automatic scanning plane tracking module configured to automatically detect the trajectory between subsequent image slices or image planes of the images acquired during the imaging of a target object.

This automatic scanning plane tracking module may be in the form of a processing unit, either of a separate processing unit or of the same processing unit of the scanning controller,
said processing unit being able to determine the relative position and/or the relative orientation of scanning planes relatively one to the other or relatively to the target object and or to a ROI of it by executing a scanning plane tracking software comprising the instructions for configuring the said processing unit to be able to identify the said position and orientation of each or of at least some of the said subsequent scanning slices or scanning planes along which the sequence of images is acquired,
said processing unit being further provided with a memory in which one or more reference scanning plane trajectories are saved and a comparator comparing the identified scanning plane trajectory followed during execution of the imaging of the target object or of a ROI of it with the said reference trajectory,
the said comparator generating information between differences in the executed scanning plane trajectory with the reference trajectory/ies and/or warning or indication iif the said differences might be relevant for the correctness of the sequence of scanning slices or scanning planes during imaging of the target object.

Tracking of the trajectory of the subsequent scanning planes may be carried out according to many different embodiments.

In one embodiment the said scanning plane trajectory tracking is carried out by tracking the probe position and orientation in a common spatial reference system in which also the target object or at least a ROI of it is placed. The position and orientation of the ultrasound probe automatically defining the position and/or orientation of each scanning slice or scanning plane along which an image is acquired.

This embodiment requires that a probe tracking system is provided in combination with the ultrasound imaging apparatus according to one or more of the said above disclosed embodiments.

According to a variant embodiment, the position and orientation of each image slice or image plane along which an image is acquired during the execution of the scanning of the target body may be determined by using the landmarks identified within each of the said images and their geometrical relationships in relation to other landmarks present in an image, such as a distance between landmarks identified in an image as well their geometrical shapes in each image.

An embodiment of the above variant comprises a landmark semantic descriptor operating in combination with the above disclosed landmark detector and a semantic description matching unit,
said semantic descriptor being a processing unit running a software comprising the instructions for the said processing unit to generate semantic descriptions univocally associated to each landmark for each or at least some of the landmarks identified in the said reference images and in the said acquired images,
said semantic descriptions matching unit being a software comprising the instructions for the said processing unit to carry out a search or a comparation between the semantic descriptions associated to the one or more landmarks present in the reference images and the semantic descriptions associated to the one or more landmarks detected in the acquired images,
and which labels an acquired image as correctly corresponding to a reference image when the relating semantic descriptions are identical one with the other and corresponding to an image acquired along an identical image slice or image plane relatively to its position and its orientation in space or relatively to the target body or to a ROI of it.

Applying the automatic semantic descriptor and the semantic descriptions matching unit to each acquired image during an imaging scan of a target body provides thus information of the correctness of the images in relation to the sequence of images defined by a certain reference scanning protocol and also information of the trajectory along which the imaging slice or the imaging plane has been displaced during the acquisition of the subsequent images of the target body.

A semantic description may comprise semantic labels describing the kind of landmark and/or geometrical labels as distances between two or more landmarks which are present in an image and/or shapes of the landmarks which are represented in an image.

According to an embodiment, the processing unit configured by software to operate as the above disclosed landmark semantic descriptor and/or the said semantic descriptions matching unit can be the same processing unit of the said scanning controller or a separate processing unit.

According to a further improvement, the correctness of the sequence of scanning slices or scanning planes executed during imaging of a target object relatively to a predefined reference sequence according to a scanning protocol of a target object may also be determined alternatively or in combination with the above embodiments and variants by
providing a machine learning algorithm which has been trained in detecting in a sequence of acquired images the presence of images taken along one or more image slices or image planes having a certain predefined position and or orientation relatively to a target body;
a processing unit being provided in which a software comprising the instruction for carrying out the said trained machine learning algorithm is or may be loaded and is or may be executed;
said processing unit having inputs for the image data of each or at least some of the images acquired along subsequent image slices or image planes during scanning of a target body ;
said processing unit having outputs for the results of the processing of the said inputted image data by the machine learning algorithm;
said results being information about the correspondence of the sequence of subsequent image slices or image planes along which the said images of the target object has been acquired with said predefined reference sequence according to a scanning protocol of a target object;
said processing unit being provided with units for signaling at least the said results and or displaying quality evaluation and/or displaying suggestions.

The above described processing unit for carrying out the said machine learning algorithm, this processing unit may be a separate one or the same processing unit of the said scanning controller or, when a separate processing unit for the landmark semantic descriptor and matching unit is provided the same processing unit of the said landmark semantic descriptor and matching unit.

In the present description the terms target body also encompasses a ROI of the said target body or a region which is imaged in relation to a certain Field of View (FOV) of the imaging apparatus.

When referring to an image slice it is meant that the said image slice is formed by at least one layer of voxels or two or more layer of voxels which are essentially parallel one to the other within the thickness of the image slice, the image slice thus having a thickness corresponding at least to the one of one voxel or of two or more adjacent voxels of respectively one of the said two or more adjacent layers of voxels.

The present invention relates also to a method for controlling the image acquisition process applied to a target body by means of an ultrasound imaging apparatus said image acquisition process comprising the steps of:
acquiring at least one ultrasound image along each one of a sequence of subsequent image acquisitions carried out each one along a different image slice or a different image plane relatively to the position and/or orientation of each of the said image slices and/or image planes with reference to a target object or a ROI of it and/or with reference one to the other of the said image slices and/or image planes;
detecting if at least some or all of the said acquired images of the said sequence of subsequent acquired images corresponds relatively to the image slice and/or the image plane along which it has been acquired with the image slice or the image plane of a predefined sequence of reference images provided in a reference image acquisition protocol of a target body or a ROI of it;
if such correspondence is found for each of the said reference images and the corresponding image slice or image plane provided in the said reference imaging protocol, defining the said image acquisition process as being carried out correctly and completely in relation to the coverage of the entire target object or a ROI of it.

In an embodiment of the said method step b) is carried out by:
identifying one or more predefined landmarks in each reference image of the sequence of subsequent reference images provided in said reference imaging protocol;
detecting one or more landmarks reproduced in at least some or all of the acquired images of the said sequence of subsequent acquired images;
for each of the said acquired images or for some of the said acquire images determining if at least one of the landmarks detected in it at step ii) corresponds to the landmarks reproduced in one of the reference images of the reference imaging protocol;
considering that a complete a correct imaging of a target object or of a ROI of it has been carried out when for all of the reference images provided in the reference imaging protocol at least an acquired image among the images of the said sequence of subsequent acquired images has been found in which the landmarks detected in it at step ii) corresponds to the landmarks reproduced in said reference image.

According to a further embodiment, the step iii) is carried out by univocally associating a semantic label to each landmark reproduced in a reference image and in an acquired image and/or at least one geometric label or more different geometric labels which labels are stored in a semantic description of a corresponding acquired image and/or reference image and by considering the an acquired image has been acquired along an image slice or an image plane which is identical to an image slice or an image plane of a reference image when the semantic descriptions associated to the said acquired image and the semantic description associated to the said reference image are matching one with the other.

According to a further feature which can be provided in any combination with the above features of the method for controlling an image acquisition process a further step is provided consisting in tracking the trajectory of the image slices and of the image planes along which the acquired images of the said sequence of subsequent acquire images has been carried out;
a further step being provided of determining the said trajectory for the image slices or the image planes along which the sequence of subsequent reference images provided in the reference imaging protocols has been generated and
of comparing the said trajectories.

According to a further embodiment of the method which can be provided in combination with one or more of the preceding embodiments or variants, the method provides additionally the following steps:
displaying at least some or all of the reference images on a display ;
highlighting a corresponding one of the reference images when an acquired image has been found corresponding to the said reference image,
continuing the steps of acquiring images and of detecting a correspondence of at least one of the said acquired images according to the method steps of one or more of the embodiment disclosed above till a corresponding acquired image has been found for each of the displayed reference images and progressively highlighting each of the displayed reference images when a corresponding acquired image has been found.

A further embodiment of the present method for controlling the image acquisition process applied to a target body by means of an ultrasound imaging apparatus said image acquisition process comprises the following steps, which can be applied alternatively by choice of the user and/or in parallel with the steps according to the one or more embodiments disclosed above:
providing a machine learning algorithm and at least one reference image acquisition process comprising a sequence of subsequent reference images of a target body taken along a plurality of image slices or image planes intersecting the said target body and/or a ROI of it and which image slices or image planes have different predetermined positions and/or orientations relatively to the target body and/or to a ROI of it and/or relatively one to the other;
training the said machine learning database in identifying, in image data acquired by carrying out a scanning of the said target body or of a ROI of it by acquiring images along a plurality of image slices or image planes intersecting the said target bidy or the said ROI and having different positions and/or orientation relative to the said target body or the said ROI and one to the other, images acquired along the image slices or the image planes defined for the reference images provided in the said reference imaging protocol;
carrying out an imaging scan of said target body and/or of said ROI of it by acquiring a sequence of subsequent images along a plurality of image slices or image planes intersecting the said target body or the said ROI which image slices or image planes have different position and orientations relatively to the said target body or the said ROI and relatively one to the other;
applying the said machine learning algorithm to the image data acquired during the said imaging scan of said target body or of a ROI of it;
said machine learning algorithm providing as a result an indication if the image data acquire during the imaging scan comprises image data corresponding to the one of the reference images provided in the reference imaging protocol.

According to an embodiment, the training of the said machine learning algorithm can be carried out according to a supervised or to an unsupervised training process.

According to a feature a supervised training of the machine learning database is carried out by
providing a training database in which each record of the said database comprises image data acquired in carrying out imaging scans of a target body or of a ROI of it in which said image data has been acquired along a plurality of image slices or image planes intersecting the said target body or a ROI of it and the information if the said image data comprises image data corresponding to the one of the reference images provided in a predetermined reference imaging protocol of a target body or a ROI of it;
for each record of the data base applying the image data acquired in the said imaging scan to the inputs of the machine learning algorithm and the information if the said acquired image data comprises image data corresponding to the one of the reference images provided in said predetermined reference imaging protocol;
and calculating the parameters of the functions of the machine learning protocol so that the inputs and the outputs fits together within a certain tolerance.

According to a further feature the training process may further comprise a testing phase in which the trained machine learning protocol is applied to image data of some of the said records of the said database by providing the said acquired image data to the input of the said trained machine learning protocol and comparing the information at the output of the machine learning algorithm with the information relating to the correspondence of the image data with the one of the reference images of the reference imaging protocol, whereby determining differences between the output of the machine learning algorithm and the information of the corresponding record of the training database and using the said difference as a parameter for further correcting the internal parameters of the functions of the machine learning algorithm. The said testing and further improving the training of the machine learning database may be carried out by applying on the differences of the calculated values at the output of the machine learning algorithm and the values provided in a record of the training data base by applying so called back propagation process.

According to an embodiment, of the above disclosed variant of the method using a machine learning algorithm, the image data relating to the acquired images in an imaging scan and the image data related to the reference images off a predetermined reference imaging protocol are in the form of raw data or of image data extracted from the said raw data or of parameters describing the pixels or voxels of each image.

According to a variant embodiment, the acquired image data and the image data of the reference images are characterized by the image slice or the image plane to which the image refers, the sad image slice and the said image plane being univocally identified by its position and its orientation in space relatively to the target body or to a ROI of it and to the image slice or the image plane of the other images of the sequence of acquired images and of the reference images, the said position and the said orientation of the said image slices and/or image planes being defined by the presence of one or more landmarks in the corresponding image and the said landmark and/or landmarks being used to code the image slice or the image plane of the acquired images and of the reference images, the said landmark or landmarks being used as the input data to the machine learning algorithm.

According to an exemplary embodiment, the said input data may be in the form of a parameter for each landmark defining either the presence or the absence in an image of the said landmark.

The above step may be carried out by considering the raw data or the image data or the parameters describing the pixels or voxels in each image, and which are related to the landmark, or the landmarks reproduced in the corresponding image.

According to an embodiment, the landmarks are each one identified by a semantic label and/or by geometric labels which are recorded as a semantic description univocally associated to the image along an image slice or an image plane and the presence or absence of a landmark in an image is carried out by using as parameters the said semantic descriptions.

In the above embodiments the reference images of the predetermined reference imaging protocol are univocally coded by the raw data or the image data or the pixel or voxel parameters relating to the one or more landmarks reproduced in the corresponding image or alternatively by a semantic landmark description comprising semantic labels identifying the one or more landmarks and /or also geometric labels such as the relative distances between landmarks reproduced in an image and/or shapes and orientation of the shapes of the landmarks.

Also the acquired images are coded by using the the raw data or the image data or the pixel or voxel parameters relating to the one or more landmarks reproduced in the corresponding image or alternatively by a semantic landmark description comprising semantic labels identifying the one or more landmarks and/or also geometric labels such as the relative distances between landmarks reproduced in an image and/or shapes and orientation of the shapes of the landmarks.

Thus the above embodiment of the method may for example provide in combination with the above disclosed steps the further steps of carrying out a landmark detection in each acquired image during the imaging scan and in each of the reference images and a step of generating semantic landmark description of each landmark, at least sone or all of the identified landmarks and/or the related semantic descriptions being univocally correlated to a corresponding image of the acquire images and of the reference images and the said landmark or landmarks or the said semantic descriptions being the input data of the machine learning algorithm, while the presence or absence of certain landmarks or of the semantic descriptions being the output data of the machine learning algorithm.

Further improvements of the invention will form the subject of the dependent claims.

### BRIEF DESCRIPTION OF THE DRAWINGS

The characteristics of the invention and the advantages derived therefrom will be more apparent from the following description of non-limiting embodiments, illustrated in the annexed drawings, in which:
Fig. 1 shows a high-level block diagram of an ultrasound image acquisition system according to an embodiment herein.
Fig. 2 is a block diagram showing an exemplary embodiment of a processing unit configured for carrying out the scanning control process according to an embodiment herein.
Fig. 3 is a graphic representation of concrete examples of the acquired images and of the landmark detection and for the checking if said acquired images corresponds to at least one or some of the sequence of reference images provided in the reference imaging protocol according to embodiments herein.
Fig. 4 is a flow diagram illustrating the steps of an embodiment of the method according to the present invention.
Fig.5 is a flow diagram illustrating the steps of a further embodiment of the method according to the present invention.
Fig. 6 diagrammatically shows the process steps of an embodiment of the method for checking correspondence of acquired images with a reference image of the sequence of reference images using semantic descriptions of landmarks detected in the said images and comparing the semantic descriptions.
Fig. 7 is a block diagram showing the step of matching the image slices or image planes of the acquired images during the imaging scan and of the reference images of the reference imaging protocol by using semantic descriptions of the landmarks detected in the said acquired and in the said reference images.
Fig. 8 and fig. 9 are diagrams describing the workflow of the method steps of providing the reference images of the reference imaging protocol with semantic descriptions of the landmarks reproduced in the said reference images and the steps of detecting landmarks in the acquired images, defining semantic descriptors of the said landmarks and carrying out the matching of the semantic descriptions associated to the said acquired images with the semantic descriptions of the reference images.
Fig. 10 shows a further embodiment of displaying the result of the imaging controller according to any of the disclosed embodiment and which can be provided in combination or alternatively to any other disclosed embodiment of showing the results of the control process.

### DETAILED DESCRIPTION OF A PREFERRED EMBODIMENT

The particular embodiments described in the figures, being by way of example and not limitative, refer to a specific diagnostic imaging sphere, that is the ultrasound one; however for a person skilled in the art it is obvious to apply his own experience in order to use such an apparatus and such a method in combination with different diagnostic imaging techniques which may present similar technical problems as the one the invention aims to solve or at least partially overcome.

Furthermore, in the explicitly disclosed example the imaging of a specific organ, namely the liver is described by acquiring a sequence of ultrasound images taken along image slices or image planes having different positions and orientation one with respect to the other and relatively to the anatomy of the liver.

This example is not to be considered limiting the field of application of the apparatus and of the method according to any of the embodiments or variants disclosed and claimed.

An ultrasound imaging scan generally comprises displacing a probe along an area of the external surface of an anatomical district of body under examination which is coincident or aligned with the organ to be scanned. Displacing the probe may require laterally shifting the probe along a linear or a curved path and/or tilting the probe backward or forward and/or rotating the probe along an axis which has at least a main directional component oriented parallel to the transmitted ultrasound beams and/or perpendicular to the said external surface.

When considering, for simplicity, a linear array probe, the probe will acquire image data along a two-dimensional slice or plane oriented parallel to the length of the linear array of ultrasound transducers and to the propagation axis of the emitted ultrasound beams.

Shifting the probe along a linear path means that the probe maintains an orientation in which the image slice or the image plane along which an image is acquired is only laterally shifted in relation to a previous image slice or image plane and in which these image slices or image planes are parallel one to the other.

Rotating the probe is defined herein as angularly displacing the probe around an axis which is parallel to the axis of the transmitted ultrasound beams, while tilting the probe backwards or forward is defined herein as angularly displacing the probe around an axis which is not parallel to the axis of propagation of the transmitted ultrasound beams.

Thus, the terms shifting, rotating and tilting when used in relation to a displacement of the probe in the present disclosure and in the claims have the meaning according to the above definitions.

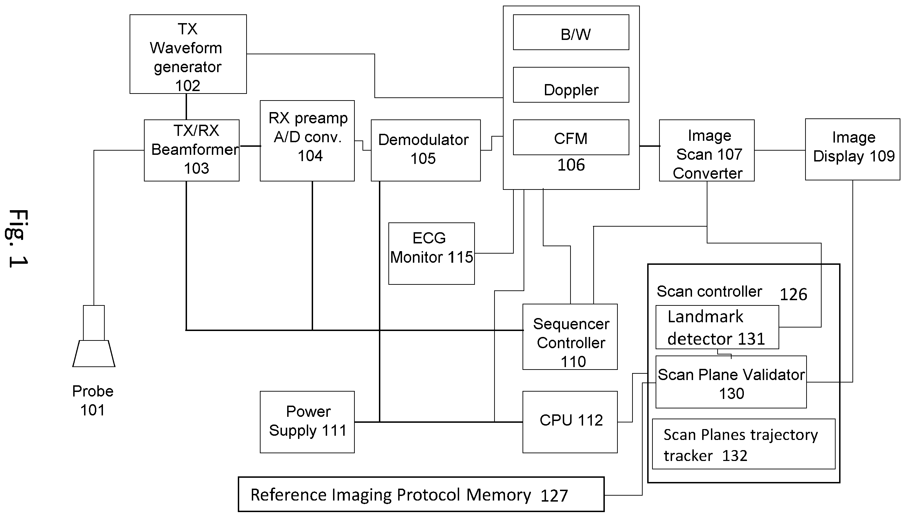

Figure 1 shows a high-level block diagram of a system for acquiring ultrasound images. This system is shown without any limiting purpose, but only for illustrative purposes to define a consolidated state of the art, wherein the ultrasound system shown is combined in a single stand-alone device.

Probe 101 can include various transducer array configurations, such as one-dimensional array, two-dimensional array, three-dimensional array, linear array, convex array and the like. Array transducers can be managed as 1D array, 1.25D array, 1.5D array, 1.75D array, 2D array, 3D array, 4D array, etc.

The ultrasound probe 101 is coupled via a wired or wireless connection to a beamformer 103. The term beamformer refers to a device for forming a beam of ultrasonic pulses, which pulses are each one generated by one of the transducers of the set of transducers which forms the probe. Since, with respect to a point arranged inside an area to be examined, the distance of each of the transducers of the probe is different and considering the speed of the acoustic wave substantially constant in the region under examination crossed by the ultrasonic pulses to reach said point, the times necessary for the various pulses generated respectively by one of the transducers of the probe, are different and to focus said pulses on the point under examination so that said pulses are constructively combined it is necessary that they reach the predefined point at the same instant and possibly with the same phase. Such process is not only typically executed in the transmission phase, but also in the reception phase. In fact, also in this case, the acoustic pulses reflected from a point inside the body under examination and for which a substantially homogeneous transit speed in said body under examination is supposed, reach the various transducers at different times due to the different length of the path between each transducer and the reflection point. In order to reconstruct the overall contribution of the reflected wave, it is therefore necessary to temporally realign the contributions of the individual pulses received by the individual transducers to obtain their constructive combination.

There are various focusing techniques in transmission and reception that allow to reduce the computational effort of this process, both as regards the necessary hardware and as regards the processing times for the formation of the transmission and/or reception beam.

The beamformer 103 according to the state of the art therefore comprises a transmission beamformer (TX) and/or a receiving beamformer (RX) which are jointly represented by the beamformer TX/RX 103. The TX and RX parts of the beamformer can be implemented together or separately. The beamformer 103 supplies transmission signals to the probe 101 and execute the beamforming of the "echo" reception signals received by the probe 101 according to the expected reception and/or transmission beam formation mode.

A TX waveform generator 102 is coupled to the beamformer 103 and generates the transmission signals which are provided by the beamformer 103 to the probe 101. The transmission signals can represent various types of ultrasonic TX signals, such as those used in connection with B-mode imaging, Doppler imaging, color Doppler imaging, pulse inversion transmission techniques, contrast-based imaging, M-mode imaging and the like. Additionally or alternatively, transmission signals can include single or multi-line transmission, transmission pulses can be focused on single lines or can be focused to extend over wider areas or an entire region of interest (called in technical jargon ROI), for example in the form of plane waves or the transmission pulses can be unfocused and made up of pulses transmitted by a single point, i.e. by a single transducer of the set of transducers at a time or by a selected subgroup of transducers or even by all the transducers of the set of transducers of the probe which are controlled in transmission so as to generate a pulse or a wave or a sequence of transmission pulses configured as if they were emitted from a common physical point of view or from multiple single points, i.e. transducers or from more subgroups of transducers.

The beamformer 103 executes beamforming on echo signals received to form reception echo signals deriving from the received signal contributions received by the individual transducers, in connection with the positions of the pixels distributed in the region of interest. For example, in accordance with certain embodiments, the transducer elements generate raw analogue reception signals which are provided to the beamformer. The beamformer adjusts the delays to focus the reception signal along one or more selected reception beams and at one or more selected depths within the region of interest (ROI). The beamformer adjusts the weighting of the received signals to achieve the desired apodization and profile. The beamformer applies weights and delays to the reception signals of the individual corresponding transducers of the probe. The delayed and weighted reception signals are then added together to form a coherent reception signal.

The beamformer 103 includes (or is coupled to) a preamplifier and/or A/D converter 104 which digitalizes the reception signals at a selected sampling frequency rate. The digitalizing process can be executed before or after the summing operation which produces the coherent reception signals. The beamformer also includes (or is coupled to) a demodulator 105 which demodulates the reception signals to remove the carrier waveform. Once the reception signals are demodulated and digitalized, complex reception signals are generated which include components I, Q (also called I, Q data pairs). The I, Q data pairs are stored in memory as image pixels. I, Q data pairs, which define the image pixels for the corresponding individual positions along the corresponding lines of sight (LOS) or lines of sight A collection of image pixels (e.g. data pairs I, Q) are collected over time and saved as 2D frames and/or 3D volumes of image data. The pixels of the image correspond to the tissues and other anatomies within the ROI.

Optionally, it is possible to program a dedicated sequence / timing controller 110 to manage the timing of the acquisition, which can be generalized as a sequence of shots aimed at selecting reflection points/targets in the ROI. The sequence controller 110 manages the operation of the TX/RX beamformer 103 in connection with the transmission of ultrasonic beams and the measurement of the image pixels in the individual LOS positions along the lines of sight. The sequence controller 110 also manages the collection of the reception signals.

One or more processors 106 and/or CPU 112 execute various processing operations as described herein.

For example, processor 106 executes a B/W module to generate B-mode images. Processor 106 and/or CPU 112 executes a Doppler module to generate Doppler images. The processor executes a Color flow module (CFM) to generate colored images. Processor 106 and/or CPU 112 may implement additional ultrasound imaging and measurement operations. Optionally, the processor 106 and/or the CPU 112 can filter the first and second displacements to eliminate motion related artifacts.

An image scan converter 107 executes image pixel scan conversion to convert the pixel format of the image from the ultrasound acquisition signal path coordinate system (e.g., the beamformer, etc.) and from the display coordinate system. For example, the scan converter 107 can convert image pixels from polar coordinates to Cartesian coordinates for frames.

A cine memory, not shown in detail, stores a timeline of frames. Frames can be stored in polar coordinate formats, Cartesian coordinates or in another coordinate system.

An image display 109 displays various ultrasound information, such as frames and information measured according to the embodiments contained herein. The display 109 shows the ultrasound image with the region of interest indicated.

A control CPU module 112 is configured to execute various tasks such as user/interface implementation and general system configuration/control. In the case of a fully software implementation of the ultrasonic signal path, the processing node usually also houses the functions of the control CPU.

A power supply circuit 111 is provided for powering the various circuits, modules, processors, memory components and the like. The power supply 111 may be an alternating current power source and/or a battery power source (e.g., in connection with portable operation).

According to the present embodiment and by way of example, the processor 106 can be associated or possibly also comprise an ECG monitoring module which receives the signals of an ECG (not shown in detail) and which allows to combine the acquisition of images with the ECG signals according to the different variants of known techniques for acquiring images synchronized by means of an ECG signal.

According to an embodiment, the CPU 112 may control the operation of a scanning controller indicated globally by 126. The scanning controller comprises a landmark detector 131 which provides for detecting in each acquired image one or more landmarks and a scan plane validator which analyses the landmarks detected in an image and validates the scan plane, i.e. the image slice or the image plane along which an ultrasound image has been acquired and reconstructed, relatively to the correspondence of the said image slice or image plane with the image slice or the image plane of a reference image of a sequence of reference images provided in a reference imaging protocol which are stored in a reference imaging protocol memory 127.

With the term image slice, a region of an object is intended having a certain thickness relatively to a planar bidimensional extension. Said thickness may comprise the thickness of a voxel or of two or more voxels. The term plane is essentially two dimensional and the image related to a plane is formed by pixels which are bidimensional unitary image elements.

The term slice may also comprise regions of space delimited by two dimensional planes which are at a distance one from the other, the distance corresponding to the thickness of one or more voxels and the two planes being parallel or not parallel one with respect to the other.

Referring to the above-described scanning or imaging controller and the related elements 130, 131 and 127, said scanning controller is configured according to embodiments herein to analyse images acquired along image slices or image planes having different positions and orientations relatively one to the other and relatively to a target object or to a ROI thereof and to detect one or more anatomical landmarks which are typical for the target object or of the RO. Once having detected the landmark or the landmarks reproduced in the acquired images, these acquired images are validated as being the ones corresponding to a correct and complete imaging protocol by determining if, among the acquired images, there are at east an image corresponding to a reference image provided by said reference imaging protocol. When for each one of the reference images provided by the reference imaging protocol there is a corresponding acquired image then the imaging process of the target object or of a certain ROI thereof is validated as being complete and correct relating to the relevant information which was expected to be gathered by the imaging session.

As it will appear clearly and with more details from the following description, validation of the acquired images may be carried out in a series of validation steps by finding for each reference image provided by the reference imaging protocol a corresponding acquired image, or said validation may be carried out by processing the entire image data set comprising each one of the images acquired in order to find if the acquired image data can be considered as comprising acquired images corresponding to each of the reference images provided by the reference imaging protocol.

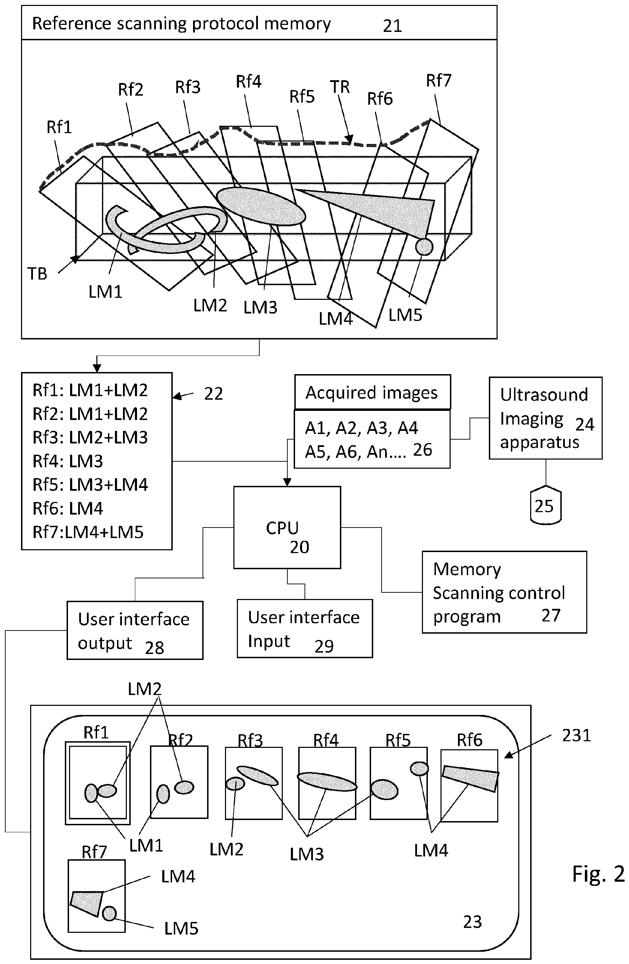

Figure 2 shows a first schematic example of the apparatus and of the method according to an embodiment in which the correctness of the scanning process for acquiring subsequent ultrasound images of a target object TB which images are acquired along several different image slices or image planes A1 to A7 is carried out progressively, during the imaging of the object TB or of a ROI thereof, by detecting one or more typical landmarks of the object TB in an acquired image and determining if the said image corresponds to a reference image RF1 to RF7 provided in a reference imaging protocol and by repeating this action for each subsequently acquired image and for each reference image.

Figure 2 is a combination of a structural block diagram showing at high level the elements of the apparatus according to an embodiment herein together with functional information related to the way of operating said structural elements.

Although figure 2 shows an embodiment in which the scanning controller is in the form of a separated processing unit, in an alternative embodiment a processing unit such as the one described in the embodiment of figure 1 and comprising a CPU 112, a Scan controller 126 and a memory for the reference imaging protocol 127 which are part of an ultrasound imaging scanner, or which is integrated in an ultrasound imaging scanner, may be used.

Coming to the processing unit of figure 2, a CPU 20 is provided in combination with one or more memories such as memory 21 for storing a reference scanning protocol and memory 27 for storing a scanning control program.

It has to be noted that these memories 21 and 27 may be configured as separate components or may also be areas of a common component, which areas are dedicated for storing specific programs and/or data such as the scanning control program and the reference scanning protocol. Further programs relating to generic control and operation of peripherals or other hardware connected and controlled by the CPU 20 may also be stored in dedicated memories and or in dedicated memory areas which are not explicitly shown in figure 2.

The CPU 20 further controls user interfaces such as output user interfaces 28 and input user interfaces 29.

The boxes 28 and 29 are intended to encompass every possible kind of interface device alternatively or in combination one with the other. Examples of output interfaces can comprise printers on different substrates like paper or films or other substrates, displays like the one explicitly shown and indicated by the numeral 23, touchscreens, acoustic outputs. Among output interfaces there might be also considered portable memories in which the output data or information may be saved.

Similarly, input interfaces may comprise keyboards, acoustic input means, gestural input means, touchscreens, point and click devices or other similar devices.

The CPU 20 also controls input of the image data generated by an ultrasound imaging apparatus 24. Image data is generated by using a probe 25 for transmitting ultrasound beams towards a target body TB or in a ROI of such body and receiving the reflected beams. With said transmitted beams a target body or a ROI of it can be scanned. Scanning can be carried out line wise, or within an image slice or an image plane along which several laterally staggered transmit beams are emitted into the target body or within a region forming a slice having a certain thickness in a direction perpendicular to the beam propagation direction.

Image slices and image planes are such that they have a certain position and certain orientation relatively one to the other and to the target body TB or a region of interest of the said target body TB.

During scanning of a target body, a sequence of images along image slices or image planes having different position and/or orientation relatively to each other and to the target body TB or to a ROI of it are acquired by displacing the probe 25 along a predefined trajectory TR. Typically the probe is displaced by hand by the radiologist or a service person skilled in operating the ultrasound scanner.

When applying ultrasound imaging for acquiring images of organs or anatomic districts of the body of human or animal beings, said organs may lay behind bone structures which renders difficult for the ultrasound beams to pass and reach the tissue of the organ to be examined. This is the case, for example, in acquiring ultrasound images of the liver. Part of the liver is covered towards the outside of the body by the rib cage so that a complete imaging scan of the liver may be obtained only by accessing the liver through different windows for the ultrasound beams provided by the morphology of the rib cage and also by displacing the probe relatively to the liver such that a predefined sequence of images is taken along predefined image slices or image planes intersecting the liver each at a certain relative position and with a certain relative orientation with respect to the liver.

The sequence of image slices or image planes along which a complete and correct imaging scan of the liver is carried out are recorded in clinical protocols which include precise scan sequences. Each image slice or image plane of a clinical protocol is identified by the presence of typical anatomical landmarks for the specific target object, in this example for the liver.

The information of the precise sequence of image slices or image planes along which images have to be acquired when carrying out the imaging of a target body TB or of a ROI of it is recorded in a so-called Reference scanning protocol in the memory 21 of the system of figure 2. An exemplary graphical representation is provided in figure 2. TB indicates the target body or a ROI of it and referring to the present example the liver or a ROI of it. The reference scanning protocol comprises a sequence of reference images indicated by Rf1, Rf2, Rf3, Rf4, Rf5, Rf6 and Rf7. Each reference image is related to a corresponding image slice or image plane intersecting the target body TB or a ROI of it and having a corresponding position and/or orientation relatively to the positions and/or orientations of the image slices or the image planes of the other reference images and relatively to the target body TB or to a ROI of it.

With LM1 to LM5, five different anatomical structures provided in the target body TB or in a ROI of it are indicated and this target structures may serve as typical anatomical landmarks which are present in the target body and which are present or not in the different reference images Rf1 to Rf7and the presence of which landmarks in a reference image univocally correlates said image with an image slice or an image plane intersecting the target body TB or a ROI of it and the landmarks.

In figure 2 this is represented by the table indicated by 22 which discloses the reference image and the landmarks which are imaged on the corresponding reference image.

According to an embodiment, the acquired images A1 to An, where n is a natural number possibly higher than 7, as indicated by 26, are submitted to processing by the CPU 20 running a scanning control program and which scanning control program contains the instructions for rendering the processing unit 20 able to do the following steps:
Detecting, in each of the acquired images, the landmarks which are present in said image;
determining if, based on the landmarks reproduced on said acquired images, one or more of said acquired images A1 to An can be considered corresponding to one or more of the reference images Rf1 to Rf7;
considering the imaging of the target body TB or of a ROI of it correct and complete if, for all the reference images, a corresponding acquired image A1 to A7 has been determined.

As it will appear more clearly by means of an exemplary embodiment, the correspondence between an acquired image and a reference image based on the landmarks reproduced on said images can be determined by using several different algorithms and methods, particularly methods which allows to determine that the image slice or the image plane of the acquired image and of the reference image are corresponding one to the other with reference to their position and/or orientation relatively to the target body TB or to a ROI of it and/or relatively to the image slices or image planes of the other images.

According to an embodiment, determining said correspondence between an acquired image and a reference image may be calculated by selecting reduced ROI containing at least one of the landmarks reproduced in the images and by carrying out an image segmentation in order to assign the pixels or the voxels to the landmark or to the tissue around said landmark and thus extracting also a shape and/or an orientation of said landmark reproduced in the images, which shape and orientation of the segmented image region related to a landmark detected on the acquired image can be compared with the shape and orientation of the segmented image region related to the landmark of the reference image.

When all the reference images Rf1 to Rf7 provided in the reference scanning protocol has been assigned a corresponding and matching image among the acquired images, the scan can be indicated as complete and correct.

According to the example of figure 2, this indication of the completeness and correctness of the scan can be signalled by printing on a display each one of the reference images RF1 to Rf7 and by highlighting, or otherwise labelling, each of said reference images in order to signal that for this reference image a matching acquired image has been found. When all the reference images Rf1 to Rf7 are highlights or labelled then the user is informed that the scan has been correctly completed and has been acquired following the clinical protocol provided for it.

The above highlighting or labelling of a reference image for which a matching acquired image has been detected is represented schematically with a double frame in relation to reference image Rf1.

According to the present exemplary embodiment, the same double frame will be applied to the other reference images Rf2 to Rf7 each time a matching acquired image is detected for one of the said reference images.

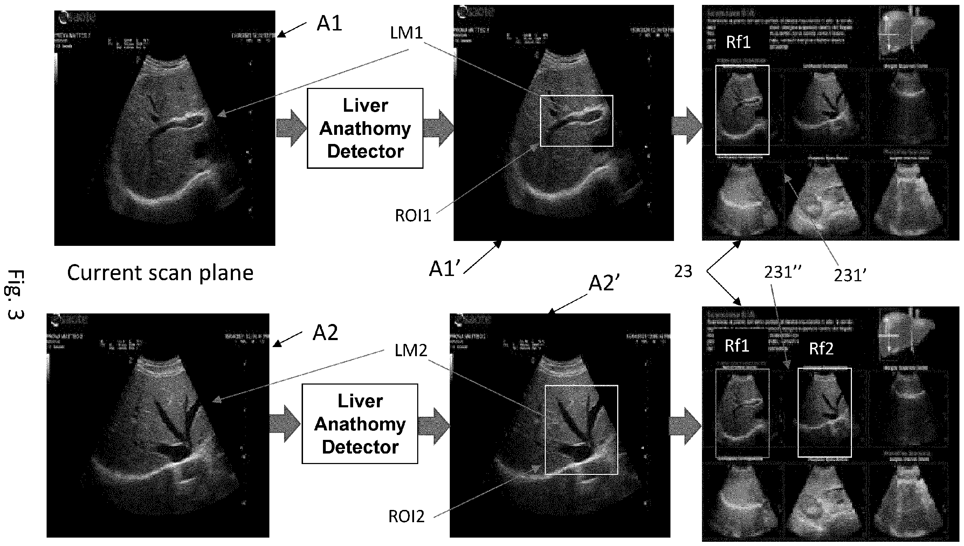

Figure 3 is an alternative representation of the above disclosed process for operating the scanning controller and determining if an imaging scan of a target body has been carried out by correctly following a predefined clinical imaging protocol.

Figure 3 shows two examples related respectively to one of two different acquired images indicated by A1 and A2. As it appears clearly, each of the two images A1 and A2 has been acquired along an image slice or an image plane having a different position and/or orientation relatively to the image slice or the image plane of the other image and relatively to the target body TB or to a ROI of it.

This fact appears evident since in the two acquired images A1 and A2, two different landmarks LM1 and LM2 are reproduced. It has to be noticed that the two different landmarks may be related to different anatomical structures within the target body or a ROI of it or to at least in part a same anatomical structure which has been imaged along a differently positioned and/or oriented image slice or image plane intersecting the said structure and thus the difference in the images A1 and A2 relates to the appearance of the landmark in the respective image A1 or A2, namely to the cross sectional shape of the structure related to the landmark along the image slice or the image plane.

The acquired images are processed by an anatomy detector 40 which processes the images A1 and A2 for identifying one or more anatomical structures which can be defined as typical anatomical landmarks of the target body TB.

The anatomy detector may be of any form such as according to the embodiment of figure 1, in which the anatomy detector is in the form of a landmark detector 131.

Generally speaking, said anatomy detector or landmark detector comprises a processing unit, which can be a separate processing unit relatively to the ultrasound imaging apparatus such as in the embodiment of figure 2 or a processing unit integrated in the ultrasound imaging system such as in the embodiment of figure 1 or it can also be the same processing unit which is provided in the ultrasound imaging apparatus for carrying out the control operations of the said apparatus and governing its functions relating to generation of the transmission beams, the receipt of the reflected beams, beamforming processes in transmission and receipt, transmitting the beam and/or receiving the beams by using specific imaging modes, decoding the received signals in order to extract the contribution of the reflectors and constructing image data and also processing the said image data in order to generate the images and display or render the images available for further processing.

The anatomy detection or the landmark detection is carried out by the processing unit, whatever embodiment of the above variants this processing unit has, by providing an anatomy or a landmark detector software which comprises the instructions for the processing unit to be able to analyse the image data or the pixels of the images or the related raw data and identify group of pixels related to the representation of an anatomical structure or a landmark.

According to an embodiment, before carrying out the anatomy or the landmark detection, the software may provide instructions for limiting said detection to certain predefined anatomical structures and/or certain predetermined landmarks, for example anatomical structures and/or landmarks which are typical for a specific target object or for a ROI of it, such as the example disclosed related to the liver as target body.

Different image processing algorithms may be coded in the instructions of the anatomy and/or landmark detector software, such as correlation algorithms of the acquired images with reference sample images and/or such as classification algorithms, among which machine learning algorithms are also possible.

As indicated by A1' and A2' in figure 3, after the processing with said anatomy detector or with said landmark detector according to the chosen variant embodiment, a landmark or an anatomical structure indicated by LM1 and LM2 is identified in the acquired image.

Said images A1' and A2' are then subjected to above disclosed step of further image processing for determining if the identified landmarks LM1 and LM2 are also represented in a reference image provided in the reference imaging protocol.

Also, this step may be carried out by using different kind of algorithms which processing steps are coded as instructions in the scanning control program 27 according to the embodiment of figure 2 or in the scan plane validator 130 according to the embodiment of figure 1.

Examples of said algorithms may comprise classification algorithms, comparation algorithms and/or other machine learning algorithms.

As it will be disclosed in the following in relation to a specific exemplary embodiment, the anatomical structures and/or landmarks detected in an acquired image provide information relatively to the kind of landmark or structure imaged and also on the position and/or orientation relatively to the target body TB or to a ROI of it of the image slice or the image plane along which said image has been acquired.

This information may be extracted from geometric data such as the shape of the area representing the anatomical structure, or the landmark reproduced in the acquired image and/or the relative position and orientation of said landmark or anatomical structure and/or of the shape relatively to other anatomical structures and/or landmarks detected in the acquired image. This allows also to determine not only if an acquired image is reproducing a landmark or an anatomical structure which is also represented in a reference image, but also which position and/or orientation has the image slice or the image plane along which the image has been acquired and if this position and/or orientation of the image slice or image plane of the acquired image is corresponding to the position and orientation of the image slice or the image plane of a reference image showing the same anatomical structure or the same landmark of the acquired one and being provided in the reference imaging protocol.

According to a further feature which is disclosed in relation to figure 3, the above processing steps, and particularly the one related to determining if the landmarks or the anatomical structures detected in an acquired image corresponds to the ones of a reference image or also if the image slice or the image plane along which an image has been acquired corresponds to the image slice or the image plane of a reference image showing at least sone of the landmarks or the anatomical structures detected in the acquired image, is preferably carried out by limiting the raw data or the image data or the image pixels to be processed to a ROI which encloses the one or more landmarks or the one or more anatomical structures detected, thereby reducing the data to be processed to the only one relevant for carrying out said process.

Further steps may also be carried out for processing the images in order to determine correspondence between acquired images and reference images according to one or more of the embodiments disclosed above which further steps may comprise also a segmentation of the images at least in relation to the ROI in which said one or more landmarks or anatomical structures detected are shown.

In figure 3, with 231' and 231" is indicated an example of the output of the apparatus relating to the results of the scan control process carried out on the acquired images and disclosed in detail with reference to the embodiment of figure 2. On the display 23, each of the reference images are displayed one beside the other. Each time an acquired image is found to be corresponding to one of the reference images displayed, this reference image is highlighted as shown for the reference image Rf1 in figure 3. Every time a further reference image among the ones displayed is found to be corresponding to an acquired image this further reference image is also highlighted as indicated by Rf2 in 231".

When the display shows that all the reference images have been highlighted then the sequence of the acquired images is considered correct and complete and corresponding to the reference imaging protocol. The user of the ultrasound system governing the probe has a clear indication if the imaging scan is completed.

Furthermore, differentiating the mode of highlighting, there is the possibility of indicating if for one the reference image only a partial correspondence has been found or if a correspondence among the acquired images has not been found at all allowing thus the user to repeat the image acquisition at least relatively to the image slices or image planes in the vicinity of the said image slice or image plane of the reference image for which no correspondence has been found with the acquired images.

According to a further feature, during the scanning of the target body TB or a ROI, it may be provided a tracker, which tracks the trajectory of one image slice or image plane with respect to the following ones during the image acquisition scan. A non-limiting and exemplary embodiment of this tracker is shown as an operating unit in figure 1 and indicated with 132. A further non-limiting embodiment is shown in figure 2, in which the tracker is implemented as a software and included in the scan control program stored in the memory 27.

In figure 2, the arrow TR shows an example of a trajectory connecting each of the reference images Rf1 to Rf7, and particularly specific reference points of said reference images, which are the same one for each image. This point or points could be any point or any pixel having a predetermined position relatively to the image slice or plane or relatively to the frames.

As an embodiment, tracking the trajectory of the image slices or the image planes along which each one of the sequence of images is acquired during an imaging scan of the target body TB can be carried out by defining on each image slice or image plane of each acquired image or of the frame of each acquired image at least one of said reference points and tracking the displacement of said at least one reference point from one image slice or image plane to the following.

Thus a further criterion to determine if an imaging scan of a target object has been carried out correctly and according to a reference imaging protocol can be to graphically determine the trajectory of the image slices or image planes of the acquired images and compare said trajectory with the one TR of the refence images Rf1 to Rf7 and this criterion may be applied alternatively or in combination with the detection of the landmarks or the anatomical structures and the matching of the said landmarks and anatomical structures with the one reproduced in the reference images according to one or more of the before disclosed embodiments.

According to an embodiment, tracking of image slice or image planes displacement one with respect to the other and/or relatively to the target body TB or to a ROI can also be carried out by applying a tracking system to the ultrasound probe and using said tracking system for calculating the trajectory of the probe in a common reference system with the target body TB or a ROI thereof. This embodiment requests additional hardware and more computational burden, but still represents a valid alternative.

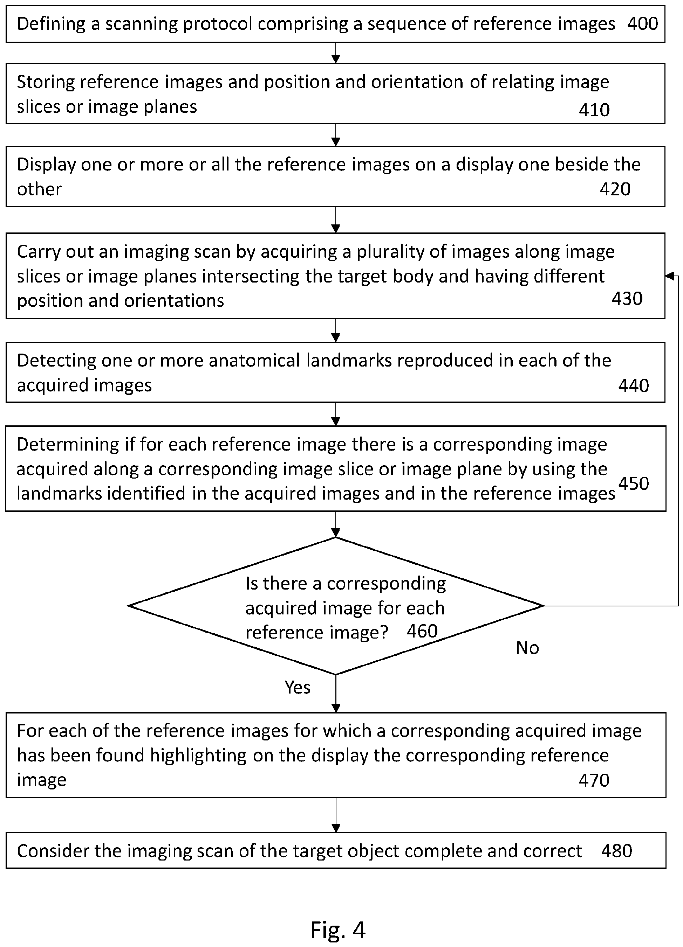

Fig. 4 is a diagram showing the workflow of the method steps according to embodiments herein, and particularly to an embodiment in which the correspondence of the acquired images with the reference images is determined one image after the other while carrying out the imaging scan by displacing the probe along the target body or a ROI thereof. While images of the target body or of the ROI are acquired one after the other along different image slices or image planes, each acquired image is processed one after the other for determining if said acquired image corresponds to one reference image of the reference imaging protocol.

The process can be carried out using any of the above disclosed embodiments and particularly by using the visual output of figure 2 and 3 in which each reference image is shown on a display and each one of these reference images is highlighted or labelled in another way when a corresponding acquired image has been detected.

As a first step 400, a scanning protocol comprising a sequence of reference images has to be defined or chosen. This may be done by using already known imaging protocols for a certain target body to be imaged, as a certain organ or anatomical district of a patient. If a known clinical scanning protocol does not exist, there is the possibility to create one by executing one or more reference imaging scans on said organ or on said anatomical district, choosing the clinical best images, determining the position and/or orientation of the imaging slices or image planes of each image and the anatomical structures or landmarks reproduced on each of said images and saving the sequence of images together with the data relating to the reproduced landmarks and/or the position and/or orientation of the image slice or image plane and other data which univocally characterize each individual acquired image as a sequence of reference images.

At step 410, the reference images of the reference imaging protocol are stored in a memory of a processing unit which can be configured according to any of the previously disclosed processing units. According to a preferred embodiment, the reference images may be stored together with the information about position and or orientation of the image slice or image plane relatively to the other image slices pr image planes of the other reference images and/o relatively to the target body or to a ROI.

One or more or all the reference images are then displayed on a display as indicated at step 420.

The apparatus is now ready for carrying out an imaging scan of the corresponding target body or a ROI thereof providing a tool for determining if the acquired images are images corresponding to a correct clinical scanning protocol or not.

At step 430, an imaging scan of the target body or of a ROI is carried out by acquiring a plurality of images along image slices or image planes intersecting the target body and having different position and orientations.

At the end of the scanning process, when all images are acquired or during scanning while the images are acquired one after the other each image is processed for detecting one or more anatomical landmarks which are reproduced in each one of the said acquired images as indicated at step 440.

As already disclosed, the detection of the anatomical landmarks can be carried out according to many different alternatives. According to an embodiment, a landmark selection is made in order to limit detection only to landmarks which are typical for the imaged target body and which provides the best information for carrying out the further steps of determining the correspondence of the acquired images with the refence images.

According to a further embodiment, landmark detection is not only limited to determining that a certain landmark is reproduced on an image, but may comprise, alternatively or in combination, analysing geometrical features, such as shape of the area of the images reproducing the landmark and its orientation and position and/or distances of a landmark from other landmarks and/or determining information about the position and/or orientation of image slice or image plane of a corresponding image relatively to the image slice or the image plane of the other images and/or relatively to the target body or to a ROI of it.

After having carried out the landmark detection at step 440 in each acquired image, a processing step 450 is carried out for determining if for each reference image there is a corresponding image acquired along a corresponding image slice or image plane by using the landmarks identified in the acquired images and in the reference images and the further information about image slice or image plane position and/or orientation.

This step 450 may be carried out according to any of the above disclosed alternative embodiments or variants.

Step 460 relates to the check if, for each reference image provided in the reference image protocol, there is a corresponding acquired image. If the answer is affirmative, step 470 is carried out by communicating to the user that the imaging scan is complete and correct. The specific step 470 provides this information according to the embodiment of figure 2, in which, for each of the reference images for which a corresponding acquired image has been found, the corresponding reference image is highlighted on the display.

Together with the visual graphic communication also a written communication may be displayed as indicated by step 480. When the acquired images are destined to be archived, a label may be applied to the imaging data signalling the fact that the image acquisition has followed correctly the clinical protocol and also the data providing evidence of that may be saved together with the images.

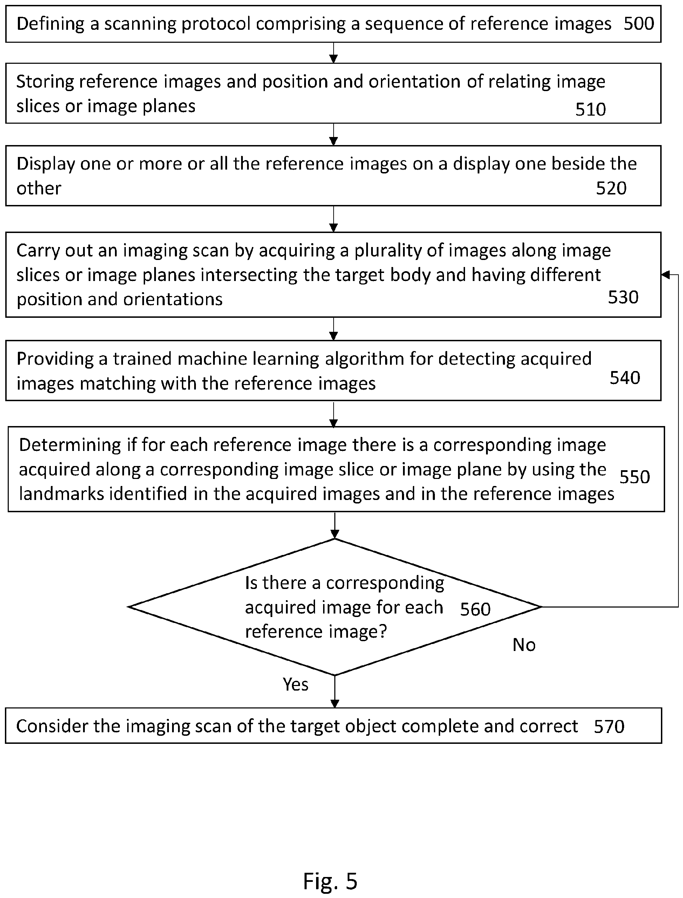

Figure 5 is a workflow diagram of an alternative embodiment which can be provided together with the previously described embodiment according to figure 4 and which can be carried out in parallel or alternatively with said embodiment of figure 4.

In this alternative embodiment, the step of determining if within the acquired mages there are acquired images corresponding to each of the reference images provided in a reference imaging protocol is not carried out image by image, but the entire data acquired is processed.

In order to carry out this kind of alternative processing, one embodiment provides for applying a machine learning algorithm which is trained in order to carry out the processing steps. The processing steps comprise determining if images acquired during an imaging scan on the same kind of target object to which the reference imaging protocol is directed match with the reference images according to the reference imaging protocol.

This can be done in different ways. According to an embodiment, the image data of the reference images of a reference imaging protocol directed to examinations of a certain target object or of a ROI are considered as a whole and are used to train a machine learning algorithm such as a classification algorithm or other kind of machine learning algorithms.

The trained machine learning algorithm is applied to the image data of the acquired images also considered as a whole and the output is at least a variable having at least two values, one relating to false and the other relating to true. Another kind of output may consider two variables one indicating if false or true is related to the determination that the acquired images are compliant with the reference images and the other if the acquired images are not compliant. This allows to have a double check on the output data. In one case when the parameter of the first output variable is true, meaning that the acquired images are compliant with the reference images, the output of the second variable should be false, meaning a negation that the acquired images are not compliant with the reference images.

According to a further variant of the above embodiment, the computation may be carried out by considering the raw data relative to the acquired and to the reference images and/or the image data calculated as a function of the said raw data and/or the parameters describing position and appearance of the pixels or voxels forming the images.

As a further feature, a preliminary step may be introduced in which step a landmark detection is carried out and optionally a landmark selection for filtering out non-interesting landmarks such as non-typical anatomical structures for the target body or structures which shape is not stable over time, or other anatomical structures which are only temporarily present in the target object.

A ROI is then determined for each landmark which has been selected and which ROI consist in a limited area of the image containing a corresponding landmark within it and being delimited by a perimetral frame of predetermined shape. Once said one or more ROI, for the said one or more landmarks, is determined, the training of the machine learning algorithm can be carried out using only the data of the reference images related to said one or more ROI and the acquired images are processed by said trained machine learning algorithm only relatively to said one or more ROI. According to a variant embodiment, the data relating to the complete acquired images is processed, so that the machine learning algorithm can carry out at the same time also the landmark detection and the ROI definition for the one or more landmarks detected. In a variant embodiment, the data related to the acquired images is previously processed for detecting one or more landmarks reproduced in it, selecting among the said detected landmarks one or more predetermined landmarks filtering out non interesting landmarks such as non-typical anatomical structures for the target body or structures which shape is not stable over time, or other anatomical structures which are only temporarily present in the target object and the trained machine learning algorithm is applied only to the one or more ROI containing the one or more selected landmarks.

Also, in the case of this embodiment providing said preliminary steps of landmark detection and optionally landmark selection and definition of one or more ROI for each of the said one or more selected landmarks, the output of the machine learning algorithm can be at least a variable having at least two values, one relating to false and the other relating to true. Another kind of output may consider two variables one indicating if false or true is related to the determination that the acquired images are compliant with the reference images and the other if the acquired images are not compliant. This allows to have a double check on the output data. In one case when the parameter of the first output variable is true, meaning that the acquired images are compliant with the reference images, the output of the second variable should be false, meaning a negation that the acquired images are not compliant with the reference images.

A further variant embodiment to which the workflow of figure 5 refers, applies the machine learning algorithm for determining for each of the said acquired images the correspondence with one of the reference images of the reference imaging protocol. This is a combination of the embodiment of the method according to figure 4 and the method providing a machine learning algorithm.

As shown in figure 5, a first step 500 provides for defining a scanning protocol comprising a sequence of reference images related to a target body under examination. This step can be limited to acquiring from third parties an already prepared clinical reference imaging protocol or it can also encompass the variant according to which said reference protocol is generated by the user according to its skills by acquiring a sequence of images during a sample imaging scan of the target body and after visual human evaluation setting these images as reliable reference images of the reference imaging protocol.

At step 510, the reference images of the reference imaging protocol are stored together with the data relating to position and/or orientation of the image slices or image planes of each reference image in a memory of the processing unit configured according to any of the previously described embodiments.

These reference images are then displayed all together one beside the other or at least a part of then on a display screen such as the one indicated by 23 and 231 in figure 2 and 3 as indicated at step 520.

Step 530 provides for carrying out an imaging scan by acquiring a plurality of images along image slices or image planes intersecting the target body or a ROI of it and having different position and orientations.

At step 540 a trained machine learning algorithm for detecting acquired images matching with the reference images is provided and applied to said acquire images.

In this embodiment, the machine learning algorithm is applied for determining if for each reference image there is a corresponding image acquired along a corresponding image slice or image plane by using the landmarks identified in the acquired images and in the reference images as indicated by step 550.

At step 560, the check is carried out for determining if there is a corresponding acquired image for each reference image. If the check is negative for at least one reference image, the scan has to be repeated. If the check is positive, then, as indicated by step 570, the acquired images are considered correctly corresponding to the above defined reference imaging protocol and can be processed and/or used for further activities.

A notice for the user that the check was negative or positive may be provided as further step.

According to an embodiment, also further steps may be provided in combination, such as the steps 470 of the previously disclosed embodiment which refers to figure 4.

As already indicated several times in the above disclosure, the steps for determining the correspondence of acquired images with the reference images may be carried out in several ways when considering the landmarks reproduced in said images.

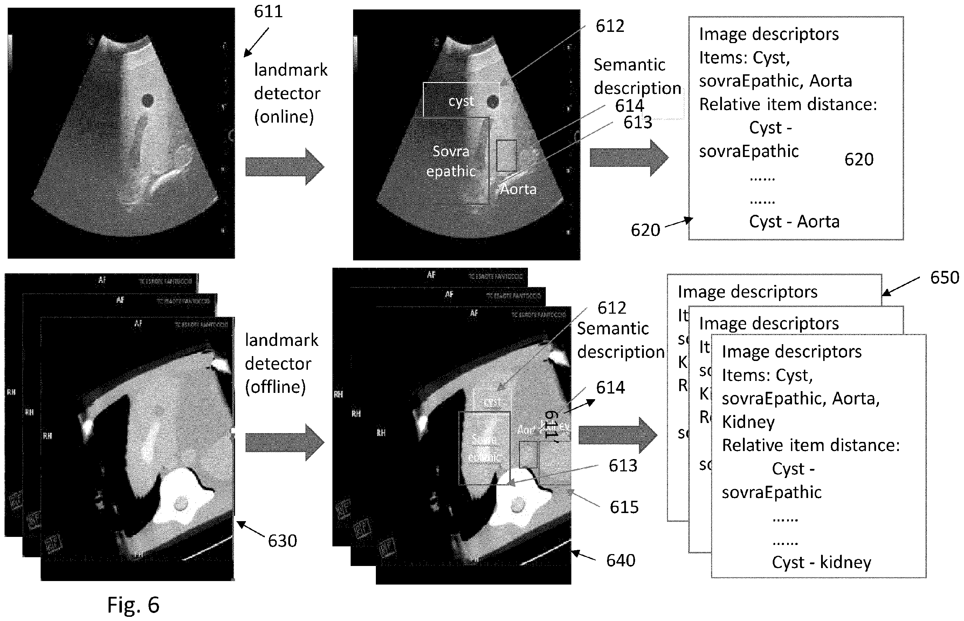

Figures 6 to 9 show an embodiment which may be useful in reducing the computational burden and speed up the processing.

This embodiment can be used in combination with any of the preceding embodiments and regards a particular way of carrying out the steps of determining the correspondence of an acquired image with a reference image.

According to a first aspect, this embodiment determines the correspondence or the identity of at least the image slice or the image plane of an acquired image with the image slice or the image plane of a reference image by carrying out a landmark detection in the reference images and in the acquired images and provides semantic descriptions for each image and each landmark or one or more landmarks or a selected group of landmarks reproduced on said images. The correspondence of an acquired image with a reference image is computed by finding, for a reference image, at least one acquired image whose semantic descriptions of the one or more landmarks reproduced on it matches with the semantic description associated to the one or more landmarks reproduced on the said reference image.

As it will appear from the following detailed description, said embodiment has a previously carried out step in which the reference images are subjected to landmark detection whereby at least one landmark or at least some of the landmarks are univocally associated to semantic descriptions of said landmarks.

The analogous steps are carried out on the acquired images in a real-time step immediately after the acquisition of the images has been terminated or during acquisition of the sequence of images.

Also the matching step of the acquired images with the reference images may be carried out immediately after termination of the image acquisitions or during the execution of the acquisition of the sequence of images.

Figure 4 shows a simplified embodiment of the process of finding correspondence of the image slices or image planes of the acquired images with an image slice or an image plane of a reference image of a reference protocol. The correspondence or identity is determined by subjecting to a matching process the semantic descriptions of the landmarks identified in one or more of the acquired images with the semantic description of a slice or a plane of a reference image. The semantic descriptors may comprise semantic labels and/or geometric labels as defined above according to the different alternatives disclosed.

The example represented in figure 4 is applied to an image acquired along a certain slice or section plane as shown by numerals 611 and to all the reference images 630. In a preparation step before carrying out any image acquisition of a target body, the reference images are processed by a landmark detector. The landmark detector identifies in each reference images the data relating to a certain landmark and the ROI within which the said landmarks are reproduced in the said reference images.

A semantic description 650 of the corresponding landmark 612, 613, 614, 615 is associated to each landmark or to one or more selected landmarks as indicated by the image 640. Further, each semantic description 650 is univocally associated to the image slice or image plane along which the reference image has been acquired. The semantic descriptions 650 may comprise semantic labels related to the corresponding landmarks and/or geometric labels related to the corresponding landmarks. Geometric labels may be any geometric feature such as the distance of a landmark from other landmarks reproduced on the same image, the shape and/or the orientation of each landmark or other geometric features.

This preparation step allows to provide the apparatus for image acquisition control according to embodiments herein with a database in which each reference image is univocally associated to a landmark description comprising semantic labels and/or one or more geometric labels for one or more of the landmarks reproduced on it.

After the preparation step is carried out, the acquisition of images of the same target object or of a ROI of it can be carried out.

From image 6 it appears clearly that each acquired image is processed by the landmark detector in order to detect one or more landmarks such as indicated by the image 611'. In this image, which is the resulting one after being processed by the landmark detector, the landmarks 612, 613 and 614, which are common to the image 650, have been detected and also the corresponding ROI. 620 indicates the semantic description comprising the semantic labels and the geometric labels related to the landmarks detected in the image 611.

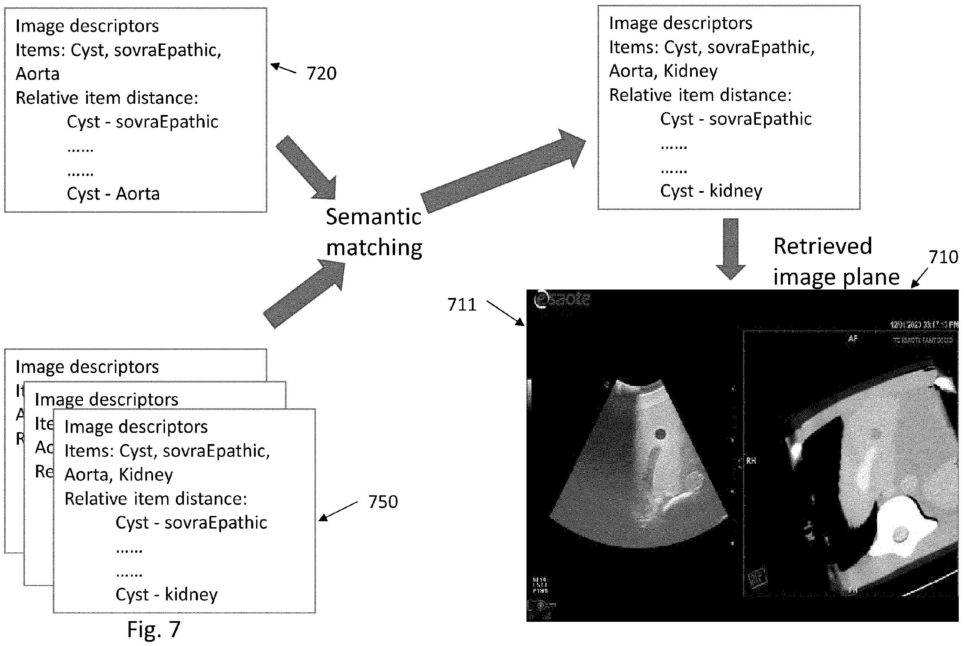

Figure 7 shows a representation of the process of determining an acquired image which is identical or corresponding to a reference image comprising a so-called semantic matching. Each acquired image, of which only one sample semantic description 720 related to only one image is shown for simplicity, is univocally associated to a semantic description such as the sample indicated by 720 and determined as disclosed in the embodiment related to figure 6.

The semantic process is carried out by determining the acquired images for which the univocally associated semantic description is corresponding or similar to the semantic description of one of the reference images indicated with 750.

When, for a reference image, a corresponding acquired image has been found, i.e. an acquired image has been found having an identical semantic description as the one associated to the said reference image, then said reference image is displayed together with a label or an indication that for this image the corresponding acquired image has been found. This label or indication may be provided according to one of the above disclosed embodiments or alternatively or in combination according to the embodiment of figure 7, in which the acquired image and the reference image associated respectively to a semantic description of the two matching semantic descriptions are displayed one beside to the other as indicated by the representation 710.

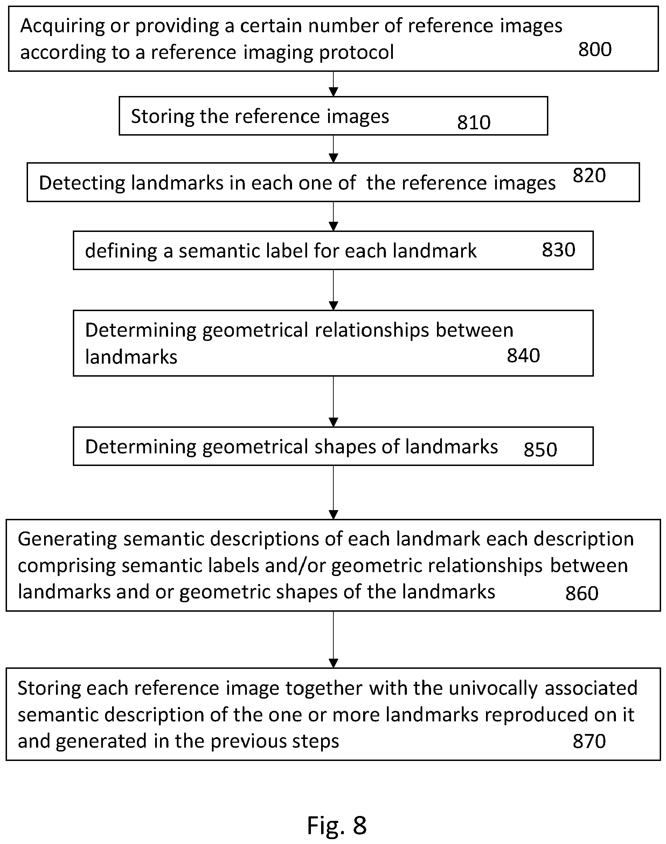

Figure 8 is a representation of an embodiment of a workflow of the process for carrying out the preparation steps of the method according to the embodiment of figure 6 and which generates, for each reference image of the reference image protocol, a univocally associated and specific semantic description comprising semantic labels and/or one or more geometric labels for the one or more landmarks reproduced on it or to only some selected landmarks thereof.

At first time a step 800 of acquiring or providing a certain number of reference images according to a reference imaging protocol is carried out. For example, an already existing imaging protocol is used and the corresponding reference images are loaded in the apparatus or rendered available for carrying out the method of the present invention.

The reference images, in the form of the corresponding raw data and/or the corresponding image data and/or the parameters describing position and aspect of the pixels or voxels of the said image may be saved as indicated by 810. At step 820, each of the said reference images or also at least only some of said reference images in one or more forms of the data representing the said reference images is subjected to a process for detecting in said images one or more landmarks. Each one or a selected ones of the said landmarks are then subjected to an operation of determining semantic labels univocally describing a quality of said landmarks and associating said semantic labels to the corresponding landmark as indicated at step 860. An optional further step 840 of determining geometric labels of the said landmarks may be carried out. One first kind of geometric labels can be the values of the distance of a certain landmark from the other landmarks identified in the image or in a certain ROI of it. Optionally, additional geometric labels can be generated and univocally associated to a landmark, which labels comprise geometric descriptions of the shape and/or of the orientation in space of the said shape of the said landmarks as indicated by step 850.

The following step 860 provides for generating image descriptions of said landmarks, said descriptions comprising semantic labels and/or geometric relationships between landmarks and/or geometric shapes of the landmarks and associating to each landmark a corresponding image description.

At step 870 each reference image together with the univocally associated semantic description of the one or more landmarks reproduced on it and generated in the previous steps are stored and are rendered available for being used in the further steps of the process of controlling the imaging scan.

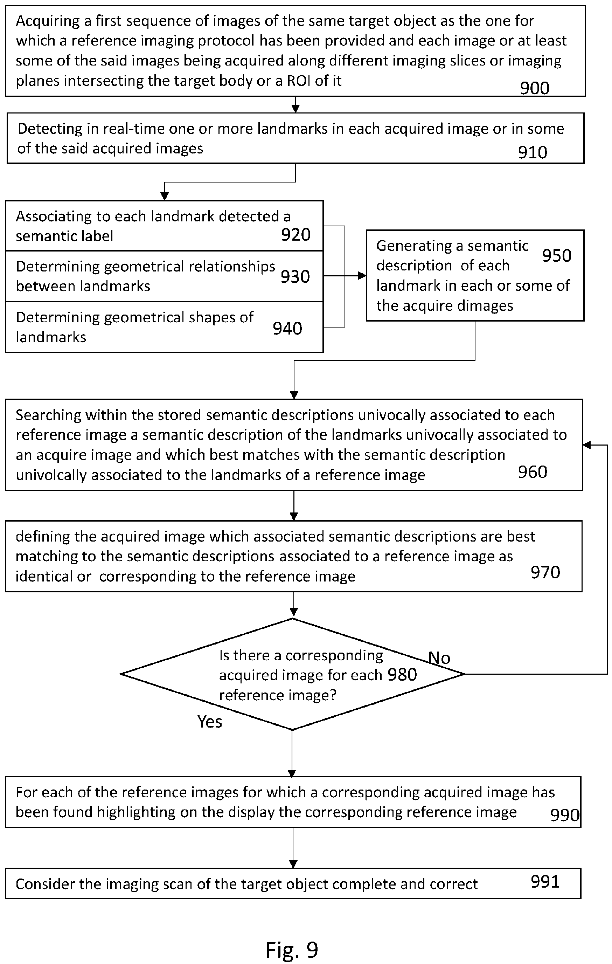

Figure 9 is a diagram showing a workflow of the process steps which are relating to said further step of the process for determining acquired images which can be considered identical or corresponding to one of the said reference images by finding images having matched semantic descriptions of the landmarks reproduced on it.

Number 900 indicates the step of acquiring a first sequence of images of the same target object as the one for which a reference imaging protocol has been provided and each image or at least some of the said images being acquired along different imaging slices or imaging planes intersecting the target body or a ROI of it. The acquired images are immediately subjected to a step of detecting landmarks withing said images as indicated by step 910. The identified landmarks are analysed and univocally associated to specific semantic labels 920 and/or geometrical labels 930 and/or geometrical shapes and/or orientations of said shapes 940 and these labels are recorded in a semantic description of said landmark at step 950. Each semantic description of the one or more landmarks detected on each or on a part of the acquired images is then univocally associated to the corresponding acquired image.

Once the semantic descriptions of each or of at least some of the acquired images have been generated this data is used for carrying out a matching process step 960 with the semantic descriptions of landmarks univocally associated to the refence images as indicated in step 960. Each acquired image which represents a best match with a reference image is defined as identical or corresponding to the matching reference image as indicated at step 970.

As already disclosed above, the operation of searching within the stored semantic descriptions univocally associated to each reference image a semantic description of the landmarks univocally associated to an acquired image and which best matches with the semantic description univocally associated to the landmarks of a reference image may be carried out only after the imaging scan has been completed by acquiring each one of the foreseen images or it can be carried out in a sort of quasi real time process in parallel with the acquisition of the sequence of foreseen images.

The example of figure 9 discloses the workflow of the variant embodiment in which the step of searching within the stored semantic descriptions univocally associated to each reference image a semantic description of the landmarks univocally associated to an acquired image and which best matches with the semantic description univocally associated to the landmarks of a reference image is carried out in a sort of quasi real time process in parallel with the acquisition of the sequence of foreseen images.

At step 980 a check is carried out if for each reference image or for at least some selected reference images a corresponding acquired image has been found according to the criteria disclosed in the previous steps. If not, the process is repeated starting from step 960.

Alternatively, it might be provided that if after a certain amount of repletion of the loop of steps 960, 970 and 980 still no correspondent acquired image has been found for a reference image, then the process may be repeated by starting from step 900 by repeating the entire or at least part of the imaging scan of the target body. Indeed, it could be possible to provide an embodiment according to which only the image acquisitions are carried out in a region of the target body corresponding to the one of the said reference images for which a matching acquired image is lacking.

If the check is positive, steps 990 and further 991 can be carried out consisting in highlighting on the display the corresponding reference image for which a corresponding acquired image has been found and considering the imaging scan of the target object complete and correct.

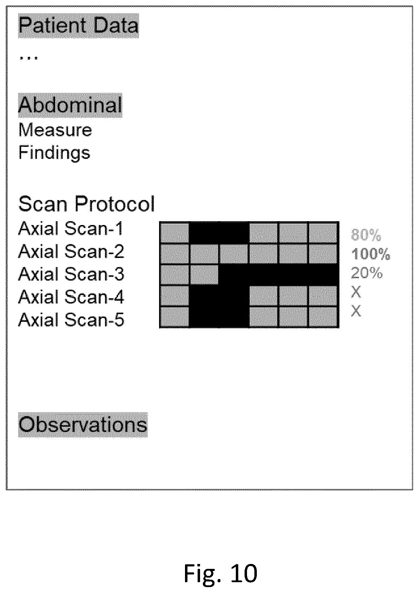

Figure 10 illustrates a further embodiment of representation of the outcome of the imaging control executed according to any of the previously disclosed examples.

Further to the personal information about a patient and to other relevant medical information such as also the kind of examination which has been carried out, the clinical protocol is indicated to be used in carrying out the imaging scan and each imaging scan is indicated. In the example five identical imaging scans have been carried out named Axial Scan-1 to Axial Scan-5. Beside each scan a table is shown which comprises a number of areas aligned on a line, each area corresponding to one image to be acquired according to the clinical protocol. Each area may be coloured in at least two different colours. Area which are coloured in black relates to reference images foreseen by the protocol and for which no corresponding acquired image has been found. The at least further colour, here grey, indicated that an acquired image was found matching with the corresponding reference image.

In the column on the right, a numerical evaluation of the quality of the scan is computed and also indications are provided relatively to the fact that some imaging scans may not be considered because such scan is in this case identical to other imaging scans already performed.

## Claims

1. An apparatus for performing diagnostic imaging examinations comprising:
an image acquisition unit;
at least one scanning controller comprising an electronic unit for operating and controlling the execution of an image acquisition protocol by means of said image acquisition unit and for carrying out image processing on at least part of the acquired images;
output units comprising at least a display for displaying one or more of the acquired images or of processed images;
said at least one electronic unit of said scanning controller being provided with a memory in which reference images of a sequence of said reference images acquired in following an ultrasound image acquisition protocol are stored, said reference images being univocally associated to one or more anatomical landmarks which are present on each of them;
said at least one electronic processing unit of said scanning controller being provided in combination with a computer program containing instructions which, when executed by said electronic unit, renders said unit able to:
run an automatic landmark detector which carries out an automatic landmark detection within each image acquired by the image acquisition unit executing said image acquisition protocol;
compare one or more landmarks detected on each one of the acquired images with the sequence of reference images and the one or more landmarks identified on the said reference images;
display each of the reference images on a display and highlight the reference images in which the one or more landmark is corresponding to the one or more landmark in the acquired images;
set the image acquisition protocol as correctly completed;
memorize the acquired images as being obtained by a correct scan for further use.

2. An apparatus according to claim 1, comprising:
a generator of a user interface which is comprised within said image acquisition unit, said user interface comprising:
one or more input units for setting signal acquisition and processing procedures for obtaining images and for setting display modes; and
one or more output units comprising at least a display for displaying graphic interface tools for communicating with a user and/or apparatus status information and/or status information about an image acquisition workflow,
said at least one electronic unit of the scanning controller being provided in combination with a computer program containing instructions which, when executed by said electronic unit,
controls the display to:
display at least some of the saved reference images;
highlight one after the other the reference images displayed when an acquired image has been found in which at least a landmark is detected corresponding to at least one landmark represented in respectively one of said reference images,
terminates the image acquisition by signalling that the image acquisition protocol has been executed correctly when, for each reference image, an acquired image has been found having at least one landmark which is identical.

3. An apparatus according to claim 1 or 2, further comprising an automatic scanning plane tracking module configured to automatically detect the trajectory between subsequent image slices or image planes of the images acquired during the imaging of a target object.

4. An apparatus according to claim 3, wherein said automatic scanning plane tracking module is in the form of a processing unit, either of a separate processing unit or of the same processing unit of the scanning controller,
said processing unit being able to determine the relative position and/or the relative orientation of scanning planes relatively one to the other or relatively to the target object and or to a ROI thereof by executing a scanning plane tracking software comprising the instructions for configuring said processing unit to be able to identify the said position and orientation of each or of at least some of the said subsequent scanning slices or scanning planes along which the sequence of images is acquired,
said processing unit being further provided with a memory in which one or more reference scanning plane trajectories are saved and a comparator comparing the identified scanning plane trajectory followed during execution of the imaging of the target object or of a ROI thereof with the said reference trajectory,
said comparator generating information between differences in the executed scanning plane trajectory with the reference trajectory/ies and/or warning or indication if said differences might be relevant for the correctness of the sequence of scanning slices or scanning planes during imaging of the target object.

5. An apparatus according to claim 3 or 4, wherein said scanning plane trajectory tracking is carried out by a probe tracking system provided in combination with the ultrasound imaging apparatus and configured to track the probe position and orientation in a common spatial reference system in which also the target object or at least a ROI thereof is placed and in which the position and orientation of the ultrasound probe automatically define the position and/or orientation of each scanning slice or scanning plane along which an image is acquired.

6. An apparatus according to any preceding claim, wherein the position and orientation of each image slice or image plane along which an image is acquired during the execution of the scanning of the target body is determined by using the landmarks identified within each of the said images and their geometrical relationships in relation to other landmarks present in an image, such as a distance between landmarks identified in an image as well their geometrical shapes in each image.

7. An apparatus according to claim 6, wherein a landmark semantic descriptor is provided operating in combination with the above disclosed landmark detector and a semantic description matching unit,
said semantic descriptor being a processing unit running a software comprising the instructions for said processing unit to generate semantic descriptions univocally associated to each landmark for each or at least some of the landmarks identified in the said reference images and in said acquired images,
said semantic descriptions matching unit being a software comprising the instructions for said processing unit to carry out a search or a comparation between the semantic descriptions associated to the one or more landmarks present in the reference images and the semantic descriptions associated to the one or more landmarks detected in the acquired images,
and which labels an acquired image as correctly corresponding to a reference image when the relating semantic descriptions are identical one with the other and corresponding to an image acquired along an identical image slice or image plane relatively to its position and its orientation in space or relatively to the target body or to a ROI thereof,
applying the automatic semantic descriptor and the semantic descriptions matching unit to each acquired image during an imaging scan of a target body to provide information of the correctness of the images in relation to the sequence of images defined by a certain reference scanning protocol and also information of the trajectory along which the imaging slice or the imaging plane has been displaced during the acquisition of the subsequent images of the target body.

8. An apparatus according to one or more of the preceding claims, wherein the correctness of the sequence of scanning slices or scanning planes executed during imaging of a target object relatively to a predefined reference sequence according to a scanning protocol of a target object is determined by providing:
a machine learning algorithm which has been trained in detecting, in a sequence of acquired images, the presence of images taken along one or more image slices or image planes having a certain predefined position and or orientation relatively to a target body;
a processing unit in which a software comprising the instruction for carrying out the said trained machine learning algorithm is or may be loaded and is or may be executed,
wherein said processing unit has inputs for the image data of each or at least some of the images acquired along subsequent image slices or image planes during scanning of a target body and outputs for the results of the processing of said inputted image data by the machine learning algorithm,
said results being information about the correspondence of the sequence of subsequent image slices or image planes along which said images of the target object have been acquired with said predefined reference sequence according to a scanning protocol of a target object,
said processing unit being provided with units for signalling at least said results and/or displaying quality evaluation and/or displaying suggestions.

9. A method for controlling the image acquisition process of a target body by means of an ultrasound imaging apparatus, said image acquisition process comprising:
a) acquiring at least one ultrasound image along each one of a sequence of subsequent image acquisitions carried out each one along a different image slice or a different image plane relatively to the position and/or orientation of each of said image slices and/or image planes with reference to a target object or a ROI thereof and/or with reference one to the other of said image slices and/or image planes;
b) detecting if at least some or all of the said acquired images of the said sequence of subsequent acquired images corresponds relatively to the image slice and/or the image plane along which it has been acquired with the image slice or the image plane of a predefined sequence of reference images provided in a reference image acquisition protocol of a target body or a ROI thereof;
c) if such correspondence is found for each of the said reference images and the corresponding image slice or image plane provided in the said reference imaging protocol, defining the said image acquisition process as being carried out correctly and completely in relation to the coverage of the entire target object or a ROI thereof.

10. Method according to claim 9, wherein step b) is carried out by:
i) identifying one or more predefined landmarks in each reference image of the sequence of subsequent reference images provided in said reference imaging protocol;
ii) detecting one or more landmarks reproduced in at least some or all of the acquired images of the said sequence of subsequent acquired images;
iii) for each of the said acquired images or for some of the said acquire images determining if at least one of the landmarks detected in it at step ii) corresponds to the landmarks reproduced in one of the reference images of the reference imaging protocol;
iv) considering that a complete/correct imaging of a target object or of a ROI thereof has been carried out when for all of the reference images provided in the reference imaging protocol at least an acquired image among the images of the said sequence of subsequent acquired images has been found in which the landmarks detected in it at step ii) corresponds to the landmarks reproduced in said reference image.

11. Method according to claim 10, wherein step iii) is carried out by univocally associating a semantic label to each landmark reproduced in a reference image and in an acquired image and/or at least one geometric label or more different geometric labels which labels are stored in a semantic description of a corresponding acquired image and/or reference image and by considering that an acquired image has been acquired along an image slice or an image plane which is identical to an image slice or an image plane of a reference image when the semantic descriptions associated to said acquired image and the semantic description associated to said reference image are matching one with the other.

12. Method according to one or more of the preceding claims 9 to 11, further comprising:
tracking the trajectory of the image slices or the image planes along which the images of said sequence of subsequent acquired images have been acquired;
determining the trajectory of the image slices or the image planes along which the sequence of subsequent reference images provided in the reference imaging protocols have been acquired; and
comparing said trajectories.

13. Method according to one or more of the preceding claims 9 to 12, further comprising:
displaying at least some or all of the reference images on a display;
highlighting a corresponding one of the reference images when an acquired image has been found corresponding to a reference image,
continuing the steps of acquiring images and of detecting a correspondence of at least one of the said acquired images till a corresponding acquired image has been found for each of the displayed reference images and progressively highlighting each of the displayed reference images when a corresponding acquired image has been found.

14. Method according to one or more of the preceding claims 9 to 13, further comprising:
providing a machine learning algorithm and at least one reference image acquisition process comprising a sequence of subsequent reference images of a target body taken along a plurality of image slices or image planes intersecting said target body and/or a ROI thereof and which image slices or image planes have different predetermined positions and/or orientations relatively to the target body and/or to a ROI thereof and/or relatively one to the other;
training said machine learning algorithm in identifying, in image data acquired by carrying out a scanning of said target body or of a ROI thereof by acquiring images along a plurality of image slices or image planes intersecting said target body or said ROI and having different positions and/or orientation relative to said target body or said ROI and one to the other, images acquired along the image slices or the image planes defined for the reference images provided in said reference imaging protocol;
carrying out an imaging scan of said target body and/or of said ROI thereof by acquiring a sequence of subsequent images along a plurality of image slices or image planes intersecting said target body or said ROI which image slices or image planes have different position and orientations relatively to said target body or said ROI and relatively one to the other;
applying said machine learning algorithm to the image data acquired during the said imaging scan of said target body or of a ROI thereof,
said machine learning algorithm providing as a result an indication if the image data acquired during the imaging scan comprises image data corresponding to the one of the reference images provided in the reference imaging protocol.

15. Method according to claim 14, wherein the training of said machine learning algorithm is carried out according to a supervised training by:
providing a training database in which each record of said database comprises:
image data acquired while carrying out imaging scans of a target body or of a ROI thereof wherein said image data has been acquired along a plurality of image slices or image planes intersecting said target body or a ROI thereof; and
the information if said image data comprises image data corresponding to the one of the reference images provided in a predetermined reference imaging protocol of a target body or a ROI thereof;
for each record of the database, applying the image data acquired in the said imaging scan to the inputs of the machine learning algorithm and the information if the said acquired image data comprises image data corresponding to the one of the reference images provided in said predetermined reference imaging protocol;
calculating the parameters of the functions of the machine learning algorithm so that the inputs and the outputs fit together within a certain tolerance.

16. Method according to claim 14 or 15, wherein the acquired image data and the image data of the reference images are **characterized by** the image slice or the image plane to which the image refers, said image slice and said image plane being univocally identified by its position and/or orientation in space relatively to the target body or to a ROI thereof and to the image slice or the image plane of the other images of the sequence of acquired images and of the reference images, the said position and the said orientation of said image slices and/or image planes being defined by the presence of one or more landmarks in the corresponding image, said landmark and/or landmarks being used to code the image slice or the image plane of the acquired images and of the reference images, the said landmark or landmarks being used as the input data to the machine learning algorithm.

17. Method according to claim 16, wherein said input data is in the form of a parameter for each landmark defining either the presence or the absence in an image of said landmark,
wherein the landmarks or at least some of the landmarks reproduced on an image are each one identified by a semantic label and/or by geometric labels which are recorded as a semantic description univocally associated to the image along an image slice or an image plane and the presence or absence of a landmark in an image is carried out by using as parameters the said semantic descriptions.

18. Method according to claim 16 or 17, further comprising:
carrying out a landmark detection in each acquired image during the imaging scan and in each of the reference images; and
generating semantic landmark descriptions of each landmark or of at least some of the identified landmarks to be used as the input data of the machine learning algorithm, while the presence or absence of certain landmarks or of the semantic descriptions is the output data of the machine learning algorithm.
